**Europäisches Patentamt**

**European Patent Office**

**Office européen des brevets**

(19)

(11) Veröffentlichungsnummer : **0 095 804**
**B1**

(12) **EUROPÄISCHE PATENTSCHRIFT**

(45) Veröffentlichungstag der Patentschrift :
19.08.87

(51) Int. Cl.⁴ : **C 12 M 3/02**

(21) Anmeldenummer : **83200720.7**

(22) Anmeldetag : **20.05.83**

(54) Verfahren und Vorrichtung zur Züchtung von Gewebezellen.

(30) Priorität : **27.05.82 CH 3264/82**

(43) Veröffentlichungstag der Anmeldung :
**07.12.83 Patentblatt 83/49**

(45) Bekanntmachung des Hinweises auf die Patenterteilung : **19.08.87 Patentblatt 87/34**

(84) Benannte Vertragsstaaten :
**BE CH DE FR GB LI LU NL SE**

(56) Entgegenhaltungen :
**EP-A- 0 050 562**
**FR-A- 1 604 693**
**FR-A- 2 358 463**
**BIOTECHNOLOGY AND BIOENGINEERING, Band 24, Nr. 9, September 1982, Seiten 2077-2086, New York, USA**

(73) Patentinhaber : **Chemap AG**
**Hölzliwisenstrasse 5**
**CH-8604 Volketswil (CH)**

(72) Erfinder : **Karrer, Daniel, Dr.**
**Blattenbach**
**CH-8636 Wald (CH)**

(74) Vertreter : **Herrmann, Peter Johannes et al**
**c/o PPS Polyvalent Patent Service AG Mellingerstrasse 1**
**CH-5400 Baden (CH)**

## Beschreibung

Die Erfindung betrifft ein Verfahren und eine Vorrichtung zur submersen Züchtung von Gewebezellen auf Mikroträgern in einem Kulturmedium.

Die Züchtung von Gewebezellen auf Trägern, sogenannten Mikrocarrier-Kulturen ist bekannt. Die Träger, auf denen die Gewebezellen wachsen, bestehen aus sehr kleinen im Kulturmedium durch sanftes Rühren in Suspension gehaltenen Teilchen. Die Zellen wachsen auf der Oberfläche dieser Mikrocarrier in einzelliger (monolayer) Schicht. Nach den bekannten Verfahren werden die Mikrocarrier separat sterilisiert, sei es in einem Autoklaven oder einem speziell dafür vorgesehenem Gefäss und nachher in den Fermenter transferiert. Die Belüftung während der Kultivation erfolgt in der Regel über einen Belüftungsring. Die anschliessende Trypsinisierung der Kultur wird in einem separaten Gefäss durchgeführt.

Nach A. L. van Wezel et al., Proc. Biochem. März 1978, S. 6 ff. ist ein Mikrocarriersystem bekannt, in welchem der Wechsel des Kulturmediums mittels eines kleinen Siebfilters aus Edelstahl mit einer Maschenweite von 60 µm erfolgt, welcher im Innern des Fermenters befestigt ist. Das Kulturmedium wird teilweise abgezogen und auf diese Weise erneuert und rezirkuliert. Durch den Einbau dieses Siebfilters werden die Mikroträger am Verlassen des Fermenters gehindert. Bei der Herstellung von menschlichen Vakzinen muss das ganze Kulturmedium abgezogen, und die Zellen müssen serumfrei gewaschen werden. Auch das sogenannte Perfusionsverfahren ist bekannt, wobei das Medium kontinuierlich gewechselt wird, indem kontinuierlich ein Teil des Kulturmediums über ein Siebfilter abgezogen und durch neues Medium ersetzt wird. Solche bekannte Siebfiltersysteme arbeiten bei kleinen Mengen und Gefässen zufriedenstellend. Sie weisen Mängel bei der Behandlung grösserer Mengen auf, wobei die Filterfläche im Vergleich zur abzutrennenden Flüssigkeit unverhältnismässig klein ist. Demzufolge verstopfen die feinmaschigen Gewebe sehr rasch, und eine Reinigung und Wiederinbetriebsetzung kann in der Regel nur nach deren Ausbau erfolgen. Auch ist das Einhängen und Befestigen des Filters schwierig, wobei eine zusätzliche Bohrung in das Kulturgefäss erfoderlich wird. Grössere Filter müssten eine weitere Befestigungsstelle haben, um nicht mit dem Mischer in Berührung zu kommen. Jeder weitere Einbau stellt wegen der dadurch auftretenden toten Ecken an die Sterlilisierung erhöhte Ansprüche. Auch ist eine Restvolumenabtrennung nach den bekannten Verfahren nicht möglich.

Die Druckschrift FR-A-2 358 463 enthält ein Verfahren und eine Vorrichtung zum Züchten von tierischen oder menschlichen Zellen. Der Behälter enthält im Boden eine Platte, die mit mehreren Bohrungen versehen ist. Durch diese Bohrungen wird das Innere des Behälters in Form von Gasblasen belüftet.

Aufgabe der Erfindung ist es, ein Verfahren und eine Vorrichtung zu schaffen, welche eine Abtrennung vom verbrauchten Substrat und eine Begasung unter optimalen Bedingungenn und Umgehung der bekannten Mängel erzielt.

Diese Aufgabe wird erfindungsgemäss nach Anspruch 1 durch ein Verfahren gelöst, bei dem das Kulturmedium alternativ vom Boden des Fermenterbehälters über eine Sinterplatte belüftet oder abgesaugt wird, und dass durch die belüftende, unter Druck stehende und durch die Sinterplatte alternativ strömende Luft diese Sinterplatte gereinigt und somit die Verstopfung der Sinterplatte verhindert wird. Das Kulturmedium im Fermenterbehälter kann somit über die Sinterplatte im Boden des Fermenters belüftet und abgezogen bzw. ausgetauscht werden, ohne dass Verluste an Zellmaterial oder Trägermaterial auftreten. Die Sintermetallplatte erfüllt somit zwei Aufgaben gleichzeitig, wobei sie gleichzeitig einen raschen Medienaustausch erlaubt und die submers zugeführte Luft regelmässig verteilt. Die Belüftung der Sinterplatte verhindert auch eine eventuelle Verstopfung derjenigen.

Gemäss einer Weiterbildung nach Anspruch 2 wird bei indirekter Begasung über einen Permeator das Kulturmedium mittels der Sinterplatte von den Miktroträgern abgetrennt und über eine Pumpe rezirkuliert.

Gemäss Anspruch 3 ist die Vorrichtung zur Durchführung des Verfahrens so ausgebildet, dass zwischen dem unteren Teil des Fermenterbehälters und einer unterhalb des Fermenterbehälters angeordneten Luftkammer im Boden des Fermenterbehälters eine Sinterplatte angeordnet ist. Diese Sinterplatte ermöglicht sowohl ein gutes und regelmässiges Belüften als auch das Absaugen des Kulturmediums aus dem Fermenterbehälter. Es ist zu berücksichtigen, dass in der Luftkammer unterhalb der Sinterplatte ein ausreichender Luftraum vorhanden ist. Die Sinterplatte besteht zweckmässig aus Keramik oder aus Metall.

Gemäss Anspruch 4 ist es zweckmässig, wenn ein Plattenpermeator über Ventile und eine Leitung mit dem Fermenterbehälter verbunden ist. Diese Anordnung verbessert weiter die Funktionsweise der Vorrichtung.

Gemäss Anspruch 5 ist es vorteilhaft, wenn der Plattenpermeator mit silikonbeschichteten flächigen Diffusionsmembranen für den Gasaustausch versehen ist.

Das Verfahren und die Vorrichtung werden anhand von Zeichnungen näher erläutert.
Es zeigen
Figur 1 ein Verfahrensschema aufgrund einer vereinfacht dargestellten Vorrichtung,
Figur 2 eine Variante des Verfahrensschemas mit einem externen Permeator,
Figur 3 einen Plattenpermeator.

Ein Fermenterbehälter 1 ist von einem Doppelmantel 2 zur Konstanthaltung der Temperatur umgebe. Ein Deckel 3 trägt ein Ankerrührwerk 4, welches von einem Elektromotor 5 angetrieben wird. Im Boden des Fermenterbehälters 1 ist eine Sinterplatte 6 eingelassen, unter welcher eine Luftkammer 7 ausgebildet ist. Ein Ventil 8 ist über einen Rohrleitungsteil 9 mit der Luftkammer 7 verbunden und führt über ein weiteres Ventil 10 auf ein Sterilluftfilter 11, welches aus einer Gasmischvorrichtung 12 gespiesen wird. In den oberen Teil des Fermenterbehälters 1 führt eine Leitung 13 zur Oberflächenbelüftung und ist an ein Sterilluftfilter 14 angeschlossen, welches mit der Gasverteilerstation 12 in Verbindung steht. Vom Deckel 3 des Fermenterbehälters 1 führt eine Leitung 15 auf ein Abluftfilter 16. Zuleitungen 17 und 18 führen aus Säure- bzw. Laugegefässen zur pH Korrektur. Das ganze Fermentersystem ist mit Mess- und Regelvorrichtungen ausgestattet. Es bedeuten die Einrichtungen $PO_2C$-Sauerstoffkontrolle, TC-Temperaturkontrolle und pHC-pH-Kontrolle. Eine Gruppe 19 ist für den Temperaturkreislauf vorgesehen. Ein Ernteventil 9' führt auf ein Ernterohr 10'.

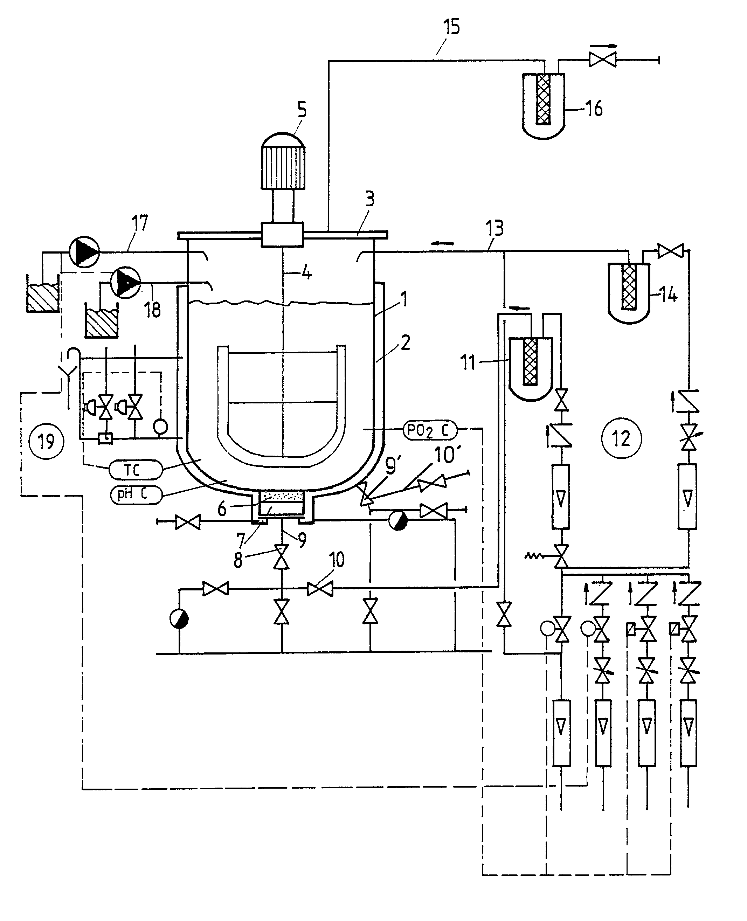

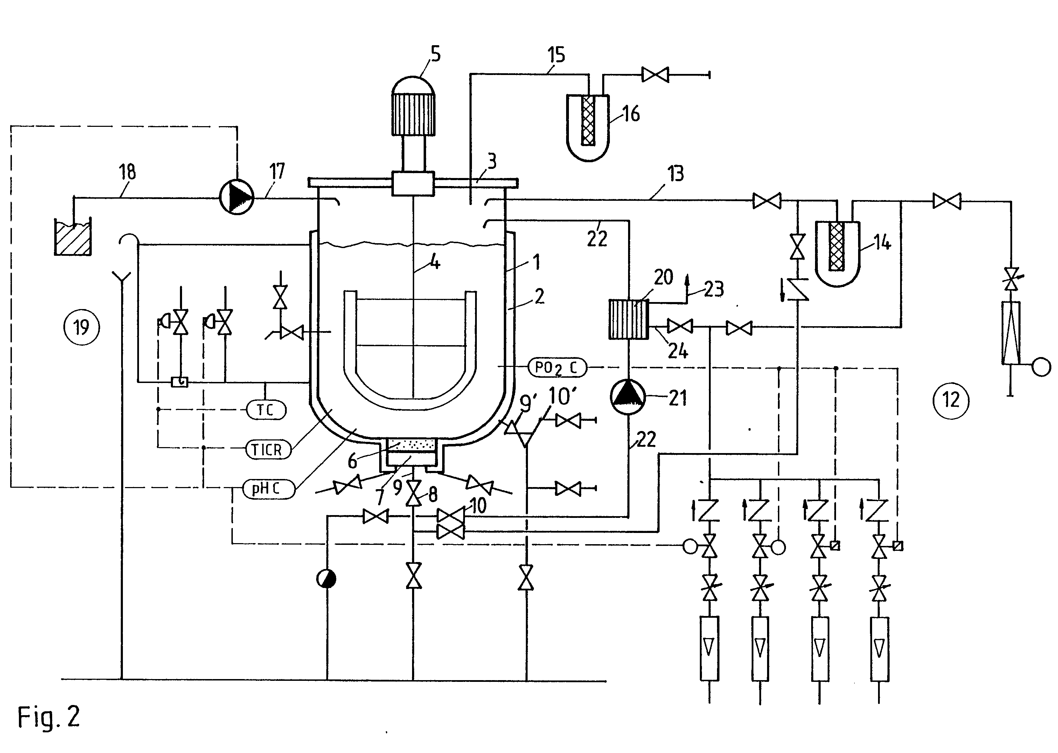

Fig. 2 unterscheidet sich von Fig. 1 im wesentlichen dadurch, dass ein Permeator 20 in einem externen Kreislauf über eine Pumpe 21 angeschlossen ist. Vom Permeator 20 führt eine Leitung 22 zurück in den Fermenterbehälter 1, eine Leitung 23 ist zur Ableitung des Überschussgases vorgesehen und eine Leitung 24 zur Zufuhr eines Gasgemisches.

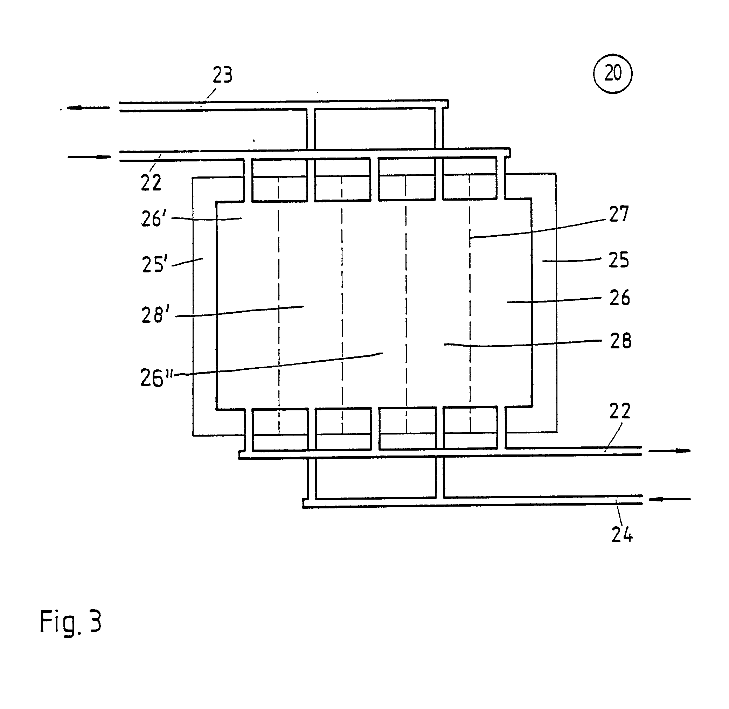

Gemäss Fig. 3 ist der Permeator 20 als Plattenpermeator ausgebildet. Der Plattenpermeator besteht aus einem Rahmen in Form eines vorderen Kammerteiles 25 und eines hinteren Kammerteiles 25', den Kammern 26, 26', 26" für das Medium. Auf den mit Filtratrinnen versehenen Kammern sind Diffusionsmembranen 27 aus reissfestem, in diesem Beispiel mit Silikon beschichtetem Material aufgebracht. Die Membranen sind auf der Flüssigkeitsseite mit Silikon beschichtet, was einen beidseitigen Gasaustausch ($O_2$, $CO_2$) durch Diffusion erlaubt. Der Permeator 20 ist mit einer Leitung 22 für den Eintritt und für den Austritt des Mediums verbunden. Eine Leitung 24 ist für den Gaseintritt und eine Leitung 23 ist für den Gasaustritt vorgesehen. Zwischen den Kammern 26, 26', 26" werden die Gaskammern 28 und 28' gebildet. Der Gasaustausch erfolgt durch Diffusion. Der Permeator 20 ist in situ dampfsterilisierbar.

Zur Inbetriebsetzung des Fermenters wird der Fermenterbehälter 1 mit 10 % des Totalvolumens an Pufferlösung (PBS = Phosphate Buffer Solution) befüllt. Auch die Mikroträger werden eingefüllt und zum Quellen gebracht. Danach wird die Suspension in bekannter Weise sterilisiert. Anschliessend wird die Pufferlösung abgelassen und der Fermenterbehälter mehrere Male mit Pufferlösung nachgespült. Nun wird sterilfiltriertes Nährmedium zusammen mit Serum bis ca. 20 % des Totalvolumens eingefüllt. Jetzt wird mit der Vorkultur in Form trypsinisierter Zellen beimpft, das Rührwerk 4 langsam während der Anwachsphase rotiert und das restliche Kulturmedium eingefüllt. Danach folgt die Züchtungsphase unter Belüftung über die Leitung 13 und/oder die Sinterplatte 6. Nach ca. 48 Stunden wird die Belüftung abgestellt und das Kulturmedium über die Sinterplatte 6 abgelassen. Danach wird Trypsinlösung eingefüllt. Der Trypsinierungsprozess wird in bekannter Weise unterbrochen und die Zellen und Träger über Ernterohr 10' transferiert.

Gemäss Fig. 2 erfolgt die Belüftung indirekt über ein Permeatorsystem ausserhalb des eigentlichen Fermenterbehälters 1. Die Flüssigkeit wird über die Sinterplatte 6 abgezogen, wobei die beiden Ventile 8 und 10 geöffnet sind und mittels der Membranpumpe 21 durch den Permeator 20 gefördert. Gleichzeitig wird Gas aus der Gasmischvorrichtung 12 über die Leitung 24 in den Permeator 20 dosiert. Das begaste Medium wird über die Leitung 22 in den Fermenterbehälter 1 zurückgeführt, während das ausgetauschte Gas über die Leitung 23 abgeführt wird.

Als besondere Vorteile des erfindungsgemässen Verfahrens und der Vorrichtung können angesehen werden, dass Sterilisation und Waschen des Trägermaterials nun im gleichen Gefäss erfolgen können, in welchem auch die Zellen gezüchtet werden. Die Sinterplatte ermöglicht einen raschen Medienaustausch und erzeugt eine hervorragende Verteilung der submers zugeführten Luft. Die Luft sorgt auch für einen zusätzlichen Reinigungseffekt der Sinterplatte. Es sind keine Eibauten in das Kulturgefäss erforderlich. Die Geometrie des Rührers kann in weiten Grenzen frei gewählt werden. Das Arbeitsvolumen ist im Bereich von 10-80 % des Totalovolumens variierbar. Auch ist kein Transfer des Trägermaterials, beispielsweise zur Trypsinisierung der Zellen erforderlich. Zudem sind Mediumaustausch und Konditionierung extern möglich.

Der externe Gasaustausch über den Permeator 20 hat weitere Vorteile. So tritt keine Schaumbildung im Fermenterbehälter 1 auf. Auch werden Scherkräfte verursacht durch aufsteigende Luftblasen im Medium verhindert. Durch die gewählten dünnen, zweckmässig silikonbeschichteten Diffusionsmembranen 27 erfolgt ein hoher Sauerstorrtransfer bei kontrollierbarer Strömung. Durch das Plattensystem kann durch Zufügen weiterer Platten der Permeator 20 ohne weiteres vergrössert werden.

**Patentansprüche**

1. Verfahren zur submersen Züchtung von Gewebezellen auf Mikroträgern in einem Kulturmedium, dadurch gekennzeichnet, dass das Kulturmedium alternativ vom Boden des Fermenterbehälters (1) über eine Sinterplatte (6) belüftet oder abgesaugt wird, und dass durch die belüftende, unter Druck stehende und durch die Sinterplatte (6) alternativ strömende Luft diese Sinterplatte (6) gereinigt und somit die Verstopfung der Sinterplatte (6) verhindert wird. (Fig. 1, 2).

2. Verfahren nach Anspruch 1, dadurch gekennzeichnet, dass bei indirekter Begasung über einen Permeator (20) das Kulturmedium mittels der Sinterplatte (6) von den Mikroträgern abgetrennt und über eine Pumpe (21) rezirkuliert wird. (Fig. 2).

3. Vorrichtung zur Durchführung des Verfahrens nach Anspruch 1, dadurch gekennzeichnet, dass zwischen dem unteren Teil des Fermenterbehälters (1) und einer unterhalb des Fermenterbehälters (1) angeordneten Luftkammer (7) im Boden des Fermenterbehälters (1) eine Sinterplatte (6) angeordnet ist. (Fig. 1, 2).

4. Vorrichtung nach Anspruch 3, dadurch gekennzeichnet, dass ein Plattenpermeator (20) über Ventile (8, 10) und eine Leitung (22) mit dem Fermenterbehälter (1) verbunden ist. (Fig. 3).

5. Vorrichtung nach Anspruch 4, dadurch gekennzeichnet, dass der Plattenpermeator (20) mit silikonbeschichteten flächigen Diffusionsmembranen (27) für den Gasaustausch versehen ist. (Fig. 3)

## Claims

1. A process for the submerged cultivation of tissue cells on micro carriers in a culture medium, characterised in that the culture medium is alternately aerated or drawn off from the base of the fermentation container (1) via a sinter plate (6), and that the aerating air being under pressure and alternately flowing through the sinter plate (6) cleans the said sinter plate (6), thereby preventing blockage of the sinter plate (6). (Figs. 1, 2).

2. A process according to claim 1, characterised in that in the case of indirect gas absorption by means of a permeator (20) the culture medium is separated from the micro carriers using the sinter plate (6) and recirculated via a pump (21). (Fig. 2).

3. Apparatus for carrying out the process according to claim 1, characterised in that between the lower portion of the fermentation container (1) and an air chamber arranged below the fermentation container (1) a sinter plate (6) is disposed in the base of the fermentation container (1). (Figs. 1, 2).

4. Apparatus according to claim 3, characterised in that a plate permeator (20) is connected to the fermentation container (1) via valves (8, 10) and a line (22). (Fig. 3).

5. Apparatus according to claim 4, characterised in that the plate permeator (20) is provided with silicon coated laminar diffusion diaphragms (27) for the exchange of gas.

## Revendications

1. Procédé de culture en submersion de cellules de tissu sur micro-supports, dans un milieu de culture, caractérisé en ce que le milieu est alternativement aéré ou aspiré du fond du fermenteur (1) par l'intermédiaire d'une plaque frittée (6), et en ce que l'air d'aération sous pression, qui traverse la plaque (6) par alternance, permet de nettoyer, donc d'éviter l'obstruction de cette plaque (6) (figures 1, 2).

2. Procédé suivant la revendication 1, caractérisé en ce que le milieu de culture, avec une aération indirecte par l'intermédiaire d'un perméateur (20), est séparé des microsupports à l'aide de la plaque frittée (6) et recyclé par l'intermédiaire d'une pompe (21) (figure 2).

3. Dispositif pour la réalisation du procédé suivant la revendication 1, caractérisé en ce qu'une plaque frittée (6) est disposée au fond du fermenteur (1), entre la partie inférieure de ce dernier et une chambre d'air (7), prévue sous ce même fermenteur (1) (figures 1, 2).

4. Dispositif suivant la revendication 3, caractérisé en ce qu'un perméateur à plaques (20) est relié au fermenteur (1), par l'intermédiaire de vannes (8, 10) et d'une conduite (22) (figure 3).

5. Dispositif suivant la revendication 4, caractérisé en ce que le perméateur à plaques (20) est muni de membranes de diffusion (27), minces et enduites de silicone, pour assurer l'échange gazeux (figure 3).

Fig. 2

0 095 804

Fig. 3